# EUROPÄISCHE PATENTANMELDUNG

(11) **EP 0 950 380 A1**
(43) Veröffentlichungstag der Anmeldung: **20.10.1999**
(21) Anmeldenummer: 99201103.1
(22) Anmeldetag: 08.04.1999
(51) Int. Cl.: A61B 19/00

(54) **Anordnung für die bildgeführte Chirurgie**

(30) Priorität: 17.04.1998 DE 19817039
(71) Anmelder: Philips Patentverwaltung GmbH, 22335 Hamburg (DE); Koninklijke Philips Electronics N.V., 5621 BA Eindhoven (NL)
(72) Erfinder: Kuhn, Michael, Dr., Röntgenstrasse 24, 22335 Hamburg (DE)
(74) Vertreter: Peuckert, Hermann, Dipl.-Ing.

(57) **Zusammenfassung**

Die Erfindung betrifft eine Anordnung für die bildgeführte Chirurgie mit einem eine Detektoranordnung (3) und eine Markeranordnung (4, 5, 6) enthaltenden optischen Positionsmeßsystem. Bei den bekannten optischen Positionsmeßsystemen, die beispielsweise zwei Kameras und jeweils drei LED's pro Markeranordnung umfassen, muß ständig eine direkte Sichtverbindung zwischen Detektoranordnung (3) und Markeranordnung (4, 5, 6), um zuverlässiges und ständiges Arbeiten des Systems zu gewährleisten und dem Chirurgen während der Behandlung ständig Aufschluß über die aktuelle Position eines mit einer Markeranordnung versehenen Behandlungsinstruments oder eines anderen Teils zu geben. Eine Unterbrechung der direkten Sichtverbindung wirkt sich negativ auf die Funktionsweise des Positionsmeßsystems aus. Die Erfindung vermeidet diese Nachteile, indem mindestens ein Spiegel (7) vorgesehen ist zur Herstellung einer indirekten Sichtverbindung (5i, 6i) zwischen der Detektoranordnung (3) und der Markeranordnung (4, 5, 6). So ist gewährleistet, daß zu jeder Zeit zumindest eine Sichtverbindung besteht.

## Beschreibung

Die Erfindung betrifft eine Anordnung für die bildgeführte Chirurgie mit einem eine Detektoranordnung und eine Markeranordnung enthaltenden Positionsmeßsystem.

Eine derartige Anordnung ist in der WO-A1-97/40763 (PHN 15.775 WO) beschrieben. Zwei Kameras, die sichtbares Licht oder Infrarotlicht detektieren können, sind ortsfest im Raum an einem Stativ angeordnet. An einem Behandlungsinstrument sind Dioden, welche von den Kameras detektierbares Licht emittieren, angebracht. Mit den Kameras kann die Position des Behandlungsinstruments in einem mit den Kameras verkoppelten Koordinatensystem bestimmt werden. Diese Position kann mittels einer vorab bestimmten Transformationsmatrix in eine Bildposition umgerechnet werden, wobei das Bild vorher beispielsweise mittels Computertomographie (CT) oder Magnetresonanztomographie (MR) erfaßt wurde. Um die genannte Transformationsmatrix zur Umrechnung von Kamerakoordinaten in Bildkoordinaten zu bestimmen, werden am Patienten während der Bilderfassung Marker angebracht, die ebenfalls abgebildet werden und im Bild sichtbar sind. Diese Marker werden anschließend mit dem mit Dioden versehenen Behandlungsinstrument oder mit einem separaten, mit Dioden versehenen Zeigeinstrument angefahren, wodurch deren Position in Kamerakoordinaten erfaßt wird. Damit ist die erforderliche Verbindung zwischen Kamerakoordinaten und Bildkoordinaten hergestellt, und die Position des Behandlungsinstruments während einer Behandlung kann im Bild angezeigt werden.
Ein solches Positionsmeßsystem wird bevorzugt in der Neurochirurgie verwendet, um dem Chirurg die aktuelle Position des Behandlungsinstruments im Gehirn des behandelten Patienten im Bild zu zeigen. Als nachteilig erweist sich jedoch bei der bekannten Anordnung, daß die direkte Sichtlinie zwischen der Detektoranordnung (den Kameras) und der Markeranordnung (den LED's) nicht unterbrochen werden darf, beispielsweise durch behandelnde Personen oder andere Geräte, um ein dauerhaftes und korrektes Arbeiten des Positionsmeßsystems zu gewährleisten.

Der Erfindung liegt deshalb die Aufgabe zugrunde, die eingangs genannte Anordnung in dieser Hinsicht zu verbessern.

Diese Aufgabe wird erfindungsgemäß dadurch gelöst, daß ein Spiegel vorgesehen ist zur Schaffung einer indirekten Sichtverbindung zwischen der Detektoranordnung und der Markeranordnung. Dadurch wird erreicht, daß zu jedem Zeitpunkt mindestens eine Sichtverbindung besteht und jederzeit die aktuelle Position der Markeranordnung bekannt ist. Der Spiegel kann dabei ortsfest gegenüber der Detektoranordnung angeordnet sein, beispielsweise fest mit der Detektoranordnung verbunden oder ortsfest an der Decke befestigt sein, oder auch verschieb- und verstellbar sein. Um auch bei einer solchen flexiblen Spiegelanordnung stets eine genaue Positionsbestimmung gewährleisten zu können, wenn nur eine indirekte Sichtverbindung über den Spiegel zwischen der Markeranordnung und der Detektoranordnung besteht, ist vorzugsweise gemäß Anspruch 2 auch am Spiegel eine Markeranordnung angeordnet zur Bestimmung der Position und Orientierung des Spiegels.

Zur Erhöhung der Betriebssicherheit trägt die vorteilhafte Ausgestaltung gemäß Anspruch 3 bei. Vorzugsweise ist die Markeranordnung so ausgestaltet, daß aus jeder Blickrichtung jeweils ausreichend viele Marker zu erkennen sind, um deren Position sicher und genau bestimmen zu können.

In einer Weiterbildung der Erfindung sind Mittel vorgesehen zum Zuordnen einer detektierten Position zu einer Markeranordnung. Diese Mittel können beispielsweise darin bestehen, daß die Marker unterschiedlich ausgestaltet sind (auf unterschiedlichen Frequenzen senden, unterschiedlich lange senden oder verschieden geometrisch angeordnet sind) und/oder zeitlich nacheinander betreibbar sind.

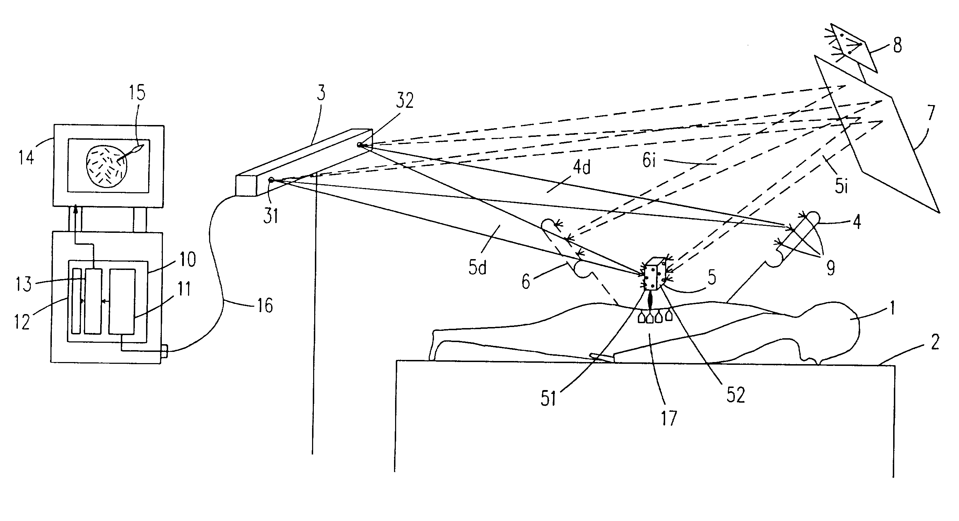

Die Erfindung wird nachfolgend anhand der Figur näher erläutert.

Der zu behandelnde Patient 1 liegt auf einem Patiententisch 2, wobei an einem Wirbelkörper 17 des Patienten 1 eine Markeranordnung 5 angebracht ist. Eine die Detektoranordnung bildende Kamera-Anordnung 3 mit zwei CCD-Flächenkameras 31,32 ist auf einem Stativ seitlich neben dem Patiententisch 2 angeordnet. Weiterhin ist ein chirurgisches Instrument 4 gezeigt, das mit drei Markern (sichtbares Licht oder Infrarotlicht aussendende Leuchtdioden) 9 versehen ist. Zwischen den Kameras 31,32 und den LED's 9 des chirurgischen Instruments 4 besteht eine direkte Sichtverbindung 4d, so daß die Position der LED's 9 im Raum und daraus die Position des chirurgischen Instruments 4 im Raum bestimmt werden kann, wozu die Detektoranordnung 3 über eine geeignete Elektronik zur stereoskopischen Vermessung von Punktlichtquellen im Raum verfügt. Die LED's 9 werden dazu geeignet angesteuert, beispielsweise in der Weise, daß sie zeitlich nacheinander kurzzeitig aufleuchten.

Auch die Markeranordnung 5 weist drei LED's 51 auf, die mit den Kameras 31,32 in direkter Sichtverbindung 5d stehen, so daß auch die Position der Markeranordnung 5 und damit die Position des Wirbelkörpers 17 bestimmt werden kann.
Um auch Positionen bestimmen zu können, wenn sich das chirurgische Instrument 4 in der gestrichelt gezeigten Position 6 befindet oder wenn die direkten Sichtlinien 4d, 5d beispielsweise durch Behandlungspersonal oder andere Geräte unterbrochen werden müssen, ist oberhalb des Patienten ein Spiegel 7 angeordnet, beispielsweise fest an der Decke montiert, über den zwischen den LED's des chirurgischen Instruments 6 bzw. der Markeranordnung 5 und den Kameras 31, 32 indirekte Sichtverbindungen 6i bzw. 5i hergestellt werden können. Die Markeranordnung 5 ist dazu auch an der dem Spiegel 7 zugewandten Seite mit Markern 52 (drei LED's) ausgestattet. Zur Positionsbestimmung des chirurgischen Instruments 6 bzw. der Markeranordnung 5 werden von den Kameras 31,32 die virtuellen Lichtpunkte der LED's im Spiegel 7 vermessen, die dann über eine geeignete Auswertelektronik in die tatsächliche Position und Orientierung des chirurgischen Instruments 6 bzw. der Markeranordnung 5 (und des Wirbelkörpers 17) umgerechnet werden. Dazu ist es erforderlich, die genaue Position und Orientierung des Spiegels 7 zu kennen. Diese können beispielsweise vorab vermessen worden sein oder immer aktuell mittels einer am Spiegel 7 angebrachten Markeranordnung 8, die in direkter Sichtverbindung mit den Kameras 31,32 steht, bestimmt werden.

Beispielsweise anhand des räumlichen Bereiches, in dem Lichtpunkte von den Kameras 31,32 detektiert werden, kann unterschieden werden, ob gerade eine virtuelle Lichtposition im Spiegel 7 oder eine tatsächliche Lichtposition einer LED bestimmt wird. Eine andere Möglichkeit wäre die sequentielle Ansteuerung der LED's am Spiegel 7, am Instrument 4 bzw. 6 und an der Markeranordnung, so daß dadurch die Zordnung definiert ist.

Wenn sowohl die Position des zu derselben LED gehörigen virtuellen Lichtpunktes als auch die Position des tatsächlichen Lichtpunktes ermittelt wurde, kann beispielsweise mittels eines geeigneten Algorithmus die tatsächliche Position mittels der aus dem virtuellen Lichtpunkt bestimmten Position verifiziert und gegebenenfalls die Meßgenauigkeit erhöht werden.

Über eine Daten- und Steuerleitung 16 ist die Detektoranordnung 3 mit einer Steuer- und Auswerteeinheit 10 verbunden. Diese umfaßt insbesondere eine Recheneinheit 11 zur Auswertung der von den Kameras 31,32 gemessenen Positionsdaten und zur Bestimmung der tatsächlichen Positionen, eine Bildspeichereinheit 12 zur Speicherung vorab ermittelter Bilddaten und eine Bildverarbeitungseinheit 13 zur Bestimmung eines Bildes, in dem jeweils die aktuelle Position des chirurgischen Instruments 4 und der Markeranordnung 5 (bzw. des Wirbelkörpers 17) eingeblendet ist. Auf einem Monitor 14 kann ein solches Bild jeweils aktuell während der Behandlung dargestellt werden; beispielhaft ist in dem Bild ein chirurgisches Instrument 15 eingeblendet.

Die gezeigte ortsfeste Anordnung des mit LED's versehenen Spiegels hat den Vorteil, daß die Detektoranordnung jederzeit beliebig im Raum verschoben werden und neu kalibriert werden kann, d.h. deren Position im Raum relativ zu den LED's des Spiegels kann jederzeit neu bestimmt werden. Es sind jedoch auch andere Anordnungen des Spiegels möglich. Es können auch mehrere Spiegel vorgesehen sein, um den Sichtbereich der Detektoranordnung noch weiter zu vergrößern und eine noch größere Betriebssicherheit zu erhalten.

Die Ausgestaltung der Detektoranordnung und der Markeranordnung hat auf die Erfindung keinen besonderen Einfluß. Es könnten statt der gezeigten zwei Zeilenkameras auch drei, jeweils eine eindimensionale Position ermittelnde Kameras verwendet werden. Auch die Anzahl und die Art der für die Markeranordnung verwendeten Marker kann anders gewählt sein (beispielsweise mehr als drei LED's pro Markeranordnung; Marker mit einem Erkennungsmuster, das von einer geeigneten Kamera oder einer anderen Detektionsvorrichtung erkannt und daraus dessen Position bestimmt wird).

## Patentansprüche

1. Anordnung für die bildgeführte Chirurgie mit einem eine Detektoranordnung (3) und eine Markeranordnung (4, 5, 6) enthaltenden optischen Positionsmeßsystem,
dadurch gekennzeichnet, daß mindestens ein Spiegel (7) vorgesehen ist zur Herstellung einer indirekten Sichtverbindung (5i, 6i) zwischen der Detektoranordnung (3) und der Markeranordnung (4, 5, 6).

2. Anordnung nach Anspruch 1,
dadurch gekennzeichnet, daß am Spiegel (7) eine Markeranordnung (8) angeordnet ist zur Bestimmung der Position und Orientierung des Spiegels (7).

3. Anordnung nach Anspruch 1,
dadurch gekennzeichnet, daß die Markeranordnung (5) mehrere derart angeordnete Marker aufweist, daß sowohl eine direkte (5d) als auch eine indirekte (5i) Sichtverbindung zur Detektoranordnung (3) besteht.

4. Anordnung nach Anspruch 1,
dadurch gekennzeichnet, daß Mittel vorgesehen sind zum Zuordnen einer detektierten Position zu einer Markeranordnung (4, 5, 6, 8).

5. Anordnung nach Anspruch 4,
dadurch gekennzeichnet, daß die Markeranordnungen (4, 5, 6, 8) unterschiedlich ausgestaltet und/oder zeitlich nacheinander betreibbar sind.
